# EUROPEAN PATENT APPLICATION

(11) **EP 4 610 938 A1**
(43) Date of publication of application: **03.09.2025**
(21) Application number: 24172899.7
(22) Date of filing: 29.04.2024
(51) Int. Cl.: G06V 10/26, A61B 8/08, G06T 7/10

(54) **DISPLAYING A MEDICAL IMAGE**

(30) Priority: 01.03.2024 US 202463560065 P
(71) Applicant: Koninklijke Philips N.V., 5656 AG Eindhoven (NL)
(72) Inventor: WEBER, Frank Michael, Eindhoven (NL); NIJHOF, Niels, Eindhoven (NL); GROTH, Alexandra, 5656AG Eindhoven (NL); VAN GEFFEN, Michel, Eindhoven (NL); FABER, Albert Louw, Eindhoven (NL); PRATER, David, Eindhoven (NL)
(74) Representative: Philips Intellectual Property & Standards

(57) **Abstract**

Proposed are schemes, solutions, concepts, designs, methods and systems pertaining to displaying a medical image of a subject. Specifically, a segmented anatomical plane image of a 3D medical image of the subject is displayed. Typically, after a user has selected a target/desired anatomical plane to be displayed for observation, the location of the anatomical plane in the 3D medical image is identified by segmenting the 3D medical image to establish anatomical context. Then, the segmented 3D medical image is processed to generate an anatomical plane image. In contrast, the invention provides that the step of segmenting the 3D medical image begins before the user selects/requests the target anatomical plane. Thus, when the user selects the target anatomical plane, the 3D medical image may already be segmented (or may already be in the process of being segmented), reducing the length of time between the request and the presentation of the anatomical plane image.

## Description

### FIELD OF THE INVENTION

The present invention relates to displaying a medical image of a subject, and more particularly to displaying an anatomical plane image.

### BACKGROUND OF THE INVENTION

During ultrasound examination, for example an examination of the heart, physicians and sonographers often want to view a particular standardized anatomical plane. These standardized anatomical planes are, however, not always easy to find quickly. One approach is therefore to start from a 3D image, establish anatomical context of the 3D image (e.g., using a segmentation algorithm), and derive the location of the desired anatomical plane in the 3D image from the established anatomical context.

A practical problem lies in the fact that the segmentation algorithm takes some time to process the image. This issue is alleviated by using a simplified segmentation algorithm, as the precision required for plane computation is typically lower than the precision required for a full quantification model. That is, some accuracy is traded for speed in order to perform a segmentation in less than a second.

Nevertheless, when applying the segmentation algorithm, it must be considered that the 3D image content changes over the course of the examination (e.g., due to a user moving the probe capturing the image). Accordingly, there will be some delay in providing the anatomical plane, even when a simplified segmentation algorithm is used. An anatomical plane of a previous image may be presented, but the information contained therein may be outdated.

There is therefore a faster means for displaying an anatomical plane of a subject.

### SUMMARY OF THE INVENTION

According to examples in accordance with an aspect of the invention, there is provided a method for displaying a medical image of a subject, comprising:
requesting a user selection, the user selection indicating a target anatomical plane of a 3D medical image of the subject;
processing, with a segmentation algorithm, the 3D medical image to generate a segmented 3D medical image;
receiving the user selection;
processing the segmented 3D medical image to generate a segmented anatomical plane image corresponding to the target anatomical plane of the user selection; and
displaying the segmented anatomical plane image,
wherein the step of processing the 3D medical image begins after requesting the user selection and before receiving the user selection.

Proposed concepts pertain to displaying a medical image of a subject. Specifically, a segmented anatomical plane image of a 3D medical image of the subject is displayed. Typically, after a user has selected a target/desired anatomical plane to be displayed for observation, the location of the anatomical plane in the 3D medical image is identified by segmenting the 3D medical image to establish anatomical context. Then, the segmented 3D medical image is processed to generate an anatomical plane image. In contrast, the invention provides that the step of segmenting the 3D medical image begins before the user selects/requests the target anatomical plane. Thus, when the user selects the target anatomical plane, the 3D medical image may already be segmented (or may already be in the process of being segmented), reducing the length of time between the request and the presentation of the anatomical plane image.

By way of explanation, during an examination of a subject (e.g., an ultrasound examination), a 3D medical image of the subject may be acquired. It is usually the case that the examiner will wish to see a standard anatomical plane of the subject (e.g., a certain cross section of the heart of the subject). However, the orientation of the 3D medical image may vary depending on the equipment used, the user conducting the examination, the position of the subject, etc. Accordingly, it may be difficult to locate the standard anatomical plane in the 3D medical image. That is, the anatomical plane may not appear in precisely the same location in each 3D medical image. As a result, the 3D medical image is usually segmented, thus providing a segmented 3D medical image having anatomical context. From the segmented 3D medical image it may be straightforward to identify an anatomical plane of interest, and cut a segmented anatomical plane image from the 3D medical image.

However, the step of segmenting the 3D medical image may take a large amount of time. Whilst this time can be reduced by using a simplified segmentation algorithm, it may still be noticeable to a user. Disclosed embodiments thus aim to reduce the noticeable impact of the time taken to display an image of the target anatomical plane of the 3D medical image.

Accordingly, it is proposed to initiate segmentation of the 3D medical image before receiving the user selection of the target anatomical plane. In practice, this may save hundreds of milliseconds of delay, making the delay less noticeable. That is, because the segmentation of the 3D medical image is (at least partially) completed before receiving a user selection, the time taken between receiving the user selection and generating (and displaying) an anatomical plane image may be reduced.

The user may thus be presented with an up-to-date anatomical plane image corresponding to a requested target anatomical plane of the subject. In turn, this may lead to improved user experience and clinical decision making.

In some embodiments, the method may further comprise a preceding step of obtaining an initial segmentation of the 3D medical image. In this case, processing the 3D medical image to generate the segmented 3D medical image may be further based on the initial segmentation of the 3D medical image.

The initial segmentation may be an approximate segmentation of the 3D medical image based on segmentations of previous 3D medical images of the subject, or a segmentation created at the start of an imaging procedure based on an initial 3D medical image of the subject. Accordingly, a subject-specific initial segmentation of the 3D medical image is obtained.

By obtaining the initial segmentation of the 3D medical image of the subject, the time to generate the segmented 3D medical image using the image segmentation algorithm may be reduced. Indeed, the initial segmentation may provide a template for completing the segmentation. In other words, the initial segmentation may start as an approximation of the segmentation of the 3D medical image, such that the segmentation algorithm may only need to modify the initial segmentation to generate the segmented 3D medical image.

Reducing the time to complete the segmentation of the 3D medical image results in a smaller delay between the user requesting/selecting a target anatomical plane, and the generation and display of the anatomical plane image.

Specifically, obtaining the initial segmentation may comprise receiving a model of a target anatomical object of the subject; and processing, with the segmentation algorithm, the 3D medical image and the model to generate the initial segmentation.

One way to provide the initial segmentation is to provide a shape (in the form of a model) of a target anatomical object that is expected to be found in the 3D medical image, and to use said shape/model to generate the initial segmentation. This will effectively create a segmentation of the 3D medical image in an approximate shape of the anatomical object found in the medical image, which may then be refined during generation of the segmented 3D medical image.

Additionally or alternatively, obtaining the initial segmentation may comprise receiving one or more previous segmented 3D medical images of the subject; and generating the initial segmentation based on the one or more previous segmented 3D medical images.

Previous segmentations of the subject may provide a good basis for future segmentations, as such segmentations are subject-specific. As this subject-specific information is used, the initial segmentation may be closer (at least in shape) to the ideal segmentation of the 3D medical image. Therefore, it may require less processing (and therefore be quicker) to generate the segmented 3D medical image using the segmentation algorithm.

In some embodiments, the method may further comprise receiving a stream of 3D medical images. In this case, the 3D medical image may be the most recent 3D medical image in the stream of 3D medical images.

In typical scenarios, 3D medical images are captured over time in an image stream, representing the anatomy of the subject changing over time. In the case of the present invention, it is an aim to process the most recent 3D medical image in the stream so that the user is presented with the most up-to-date view of the anatomy of the subject.

The most recent 3D medical image may also be the most recent 3D medical image having a certain anatomy, or a certain anatomy in a given state. For example, the most recent 3D medical image may relate to a defined heart phase within the most recent heartbeat acquired in the stream. More specifically, the most recent 3D medical image may be the latest end-diastolic, mid-systolic, or end-systolic 3D medical image.

The stream of 3D medical images may be received in real-time.

In this case, it may be even more important that the segmentation of the 3D medical image is performed before the user request is received in order to reduce a time between the time the real-time 3D medical image is received, and the anatomical plane image is displayed.

In some embodiments, the method may further comprise increasing computational resources available to process the 3D medical image with the segmentation algorithm.

In 3D imaging, the system generating the images dedicates a significant amount of computational resources to perform the imaging. However, it may be beneficial to reduce some of the computational resources allocated to rendering the 3D medical image with minimal impact on the image quality. Reducing these computational resources means that more computation resources are available for performing the segmentation by the segmentation algorithm. As a result, the time taken to perform the segmentation may be reduced. There may thus be a further reduced delay between the user requesting/selecting a target anatomical plane, and the generation and display of the anatomical plane image.

Furthermore, processing the segmented 3D medical image to generate the segmented anatomical plane image may comprise identifying a 2D cross section of the segmented 3D medical image including the target anatomical plane of the user selection; and extracting the segmented anatomical plane image from the segmented 3D medical image based on the identified 2D cross section.

This is one way in which to process the segmented 3D medical image to obtain the segmented anatomical plane image. Indeed, from the segmented 3D medical image it should be straightforward to identify the 2D cross section including the target anatomical plane (as the segmentation provides anatomical context to the 3D medical image). Then, the segmented anatomical plane image, which is two dimensional, may be extracted using any known method for taking a 2D cut of a 3D image.

In specific embodiments, requesting the user selection may comprise displaying a plurality of anatomical sketches of target anatomical planes included in the 3D medical image. Receiving the user selection may comprise receiving a selection of one of the anatomical sketches from the user.

In typical systems, representative sketches of target anatomical planes may be presented to the user for straightforward and intuitive selection of the target anatomical plane. The selection may thus comprise detecting which of the anatomical sketches are selected (e.g., they may be touched, clicked, or a button corresponding to the target anatomical image may be activated).

Some embodiments may also comprise scanning the subject to acquire the 3D medical image.

Of course, in some embodiments the method may also comprise performing the scan of the subject to acquire the 3D medical image, or a stream of 3D medical images including the 3D medical image. Nevertheless, the method may simply receive the 3D medical image, or obtain the 3D medical image from a database (i.e., does not require direct scanning of the subject).

The 3D medical image may comprise a 3D ultrasound image of the subject. A 3D ultrasound examination may particularly benefit from the present invention due to the variation in anatomy over time of anatomical objects typically imaged using ultrasound, as well as the variety by which the user can perform the examination (e.g., place and orient the probe). Nevertheless, the invention is not restricted hereto, and may also apply to other medical imaging modalities.

According to examples in accordance with a further aspect of the invention, there is provided a computer program comprising computer program code means adapted, when said computer program is run on a computer, to implement a disclosed method of displaying a segmented medical image of a subject.

According to additional examples in accordance with an aspect of the invention, there is provided a system for displaying a medical image of a subject, comprising:
an interface configured to:
request a user selection, the user selection indicating a target anatomical plane of a 3D medical image of the subject; and
receive the user selection; and
a processor configured to:
   process, with a segmentation algorithm, the 3D medical image to generate a segmented 3D medical image;
   process the segmented 3D medical image to generate a segmented anatomical plane image corresponding to the target anatomical plane of the user selection,
   wherein processor is configured to begin processing the 3D medical image after the interface requests the user selection and before the interface receives the user selection, and
   wherein the interface is further configured to display the segmented anatomical plane image.

In some embodiments, the processor may be further configured to obtain an initial segmentation of the 3D medical image. In this case, the processor may process the 3D medical image to generate the segmented 3D medical image further based on the initial segmentation of the 3D medical image.

In addition, the processor may be further configured to increase computational resources available to process the 3D medical image with the segmentation algorithm.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 depicts a typical prior art workflow of a medical examination, including display of a target anatomical plane image;
Fig. 2 depicts a workflow of a medical examination, including display of a target anatomical plane image, according to an exemplary embodiment of the invention;
Fig. 3 presents a flow diagram of a method for displaying a medical image of a subject according to an embodiment of the invention;
Fig. 4 presents a flow diagram of a method for displaying a medical image of a subject according to another embodiment of the invention;
Fig. 5 presents a block diagram of a system for displaying a medical image of a subject according to a further embodiment of the invention;
Fig. 6 provides a simplified block diagram of a computer within which one or more parts of an embodiment may be employed.

### DETAILED DESCRIPTION OF THE EMBODIMENTS

The invention will be described with reference to the Figures.

It should be understood that the Figures are merely schematic and are not drawn to scale. It should also be understood that the same reference numerals are used throughout the Figures to indicate the same or similar parts.

It should also be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. The mere fact that certain measures are recited in mutually different dependent claims does not indicate that a combination of these measures cannot be used to advantage.

Proposed are schemes, solutions, concepts, designs, methods and systems pertaining to displaying a medical image of a subject. Specifically, a segmented anatomical plane image of a 3D medical image of the subject is displayed. Typically, after a user has selected a target/desired anatomical plane to be displayed for observation, the location of the anatomical plane in the 3D medical image is identified by segmenting the 3D medical image to establish anatomical context. Then, the segmented 3D medical image is processed to generate an anatomical plane image. In contrast, the invention provides that the step of segmenting the 3D medical image begins before the user selects/requests the target anatomical plane. Thus, when the user selects the target anatomical plane, the 3D medical image may already be segmented (or may already be in the process of being segmented), reducing the length of time between the request and the presentation of the anatomical plane image.

Overall, disclosed concepts aim to reduce a time between a user selecting a target anatomical plane to be visualized, and display of an image of the target anatomical plane. This is achieved by reducing a time taken to analyze the medical image to identify a location of the target anatomical plane. Specifically, the time taken for segmentation of the 3D medical image, between the user selection of the target anatomical plane and display of the anatomical plane image, is reduced. Various concepts are proposed to achieve this aim, such as initiating segmentation of the 3D medical image before receiving the user selection of the target anatomical plane, but after requesting the selection (i.e., when a menu is shown, but before the user selects the target anatomical plane from the menu). Ideally, segmentation will be completed before the user selects the target anatomical plane. Nevertheless, to reduce the amount of time taken by the segmentation, an initial segmentation may be obtained (e.g., generated from a dynamic model to represent the anatomy of the subject, or generated based on previous segmentations of the subject) that can be used to base the segmentation of the 3D medical image (i.e., as a starting point for the segmentation algorithm). Furthermore, increased computational resources may be allocated to the segmentation algorithm (e.g., by reducing computational resources used for capturing future 3D medical images).

By way of explanation, during a medical imaging session (e.g., an ultrasound examination of the heart) the examiner often wishes to observe standardized views/anatomical planes (e.g., of a certain cross section of and/or component of the heart). The standardized views are not always simple to find within a 3D medical image, for example due to differences in probe location or positioning of the subject. One approach is therefore to obtain the 3D medical image, establish anatomical context by segmenting the 3D medical image, and from the segmented 3D medical image (which will have described anatomical context by virtue of the segmentation) derive the location of the desired view.

However, segmentation algorithms (even when simplified) take a noticeable length of time to process 3D medical images. This problem is particularly noticeable when the content in the present 3D medical image compared to previous 3D medical images in an image stream changes (e.g., due to repositioning of the probe). This will require the identification of the location of the desired anatomical plane, and therefore a segmentation procedure, and thus a delay to a user request for the desired anatomical plane. Otherwise, the previous information will be outdated (i.e., will relate to when the probe was in a different position).

To mitigate this issue, disclosed embodiments propose a multi-stage approach:
(i) Prior to the user requesting/selecting a target anatomical plane, and even before requesting the user to select a target anatomical plane, an initial segmentation may be obtained. This initial segmentation may be generated for the whole target anatomy. The initial segmentation may be used as the starting point of the later adaptation, thus reducing a time to complete segmentation of the 3D medical image;
(ii) Segmentation of the latest 3D medical image begins already when a request for a user selection (of a target anatomical plane) is communicated. For example, the segmentation begins when an anatomical plane selection menu is opened and/or displayed to the user. This provides extra computation time while the user selects the target anatomical plane; and
(iii) Computing resources available to the segmentation algorithm are increased whilst performing the segmentation.

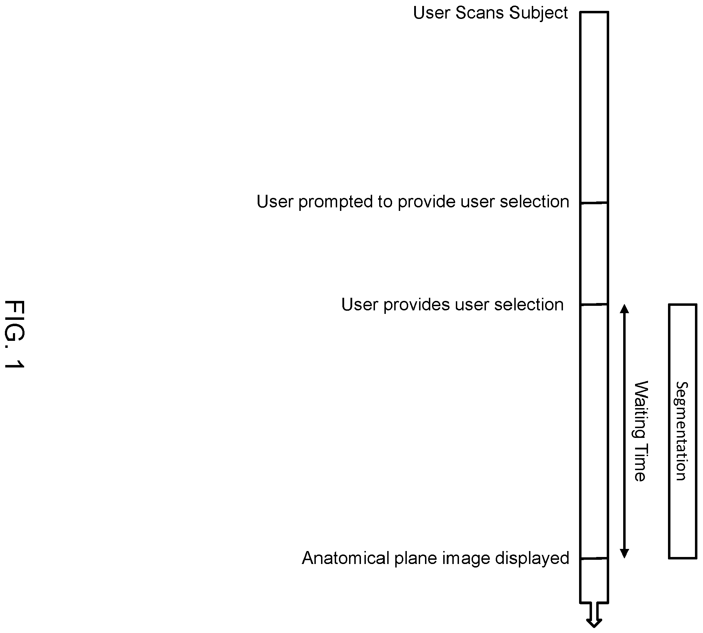

Fig. 1 depicts a typical prior art workflow of a medical examination, including display of a target anatomical plane image.

As shown, the workflow begins with a user (e.g., an examiner or sonographer) scanning the subject. They may capture a single 3D medical image, or may capture a stream of 3D medical images (i.e., a 3D medical video). At a certain point during the workflow, the user either opens an anatomical plane selection dialog box (or equivalent), or the user is prompt to select a target anatomical plane. Then, after a period of time, the user selects the target anatomical plane to be visualized on a screen.

At that moment, the latest 3D medical image of the stream of 3D medical images is taken (or the latest 3D medical image corresponding to a target phase of the imaged anatomy), and is processed to generate a 2D anatomical plane image corresponding to the target anatomical plane selected by the user. To this end, the 3D medical image is segmented, with a segmentation algorithm, to generate a segmented 3D medical image. The segmented 3D medical image is then processed to locate the target anatomical plane within the 3D medical image, and to generate the anatomical plane image (e.g., by taking a cross section of the 3D medical image). However, the step of segmenting the 3D medical image will take up the majority of the time. There may be additional processing steps not depicted here, such as post-processing to improve a quality of the anatomical plane image, or a labelling of the anatomical plane image based on the segmentation. Finally, the anatomical plane image is displayed to the user.

Furthermore, once the target anatomical plane is located to generate the anatomical plane image, subsequent 3D medical images may be processed based on the same location to generate subsequent anatomical plane images as long as the probe acquiring the subsequent 3D medical images remains in the same place. In other words, as long as the probe remains in the same location throughout acquisition of a 3D medical image stream, the result of identifying the target anatomical image plane can be used on the 3D medical image stream to acquire a stream of anatomical plane images.

As seen, there may be significant waiting time between the user selecting the target anatomical plane, and the anatomical plane image being displayed to the user.

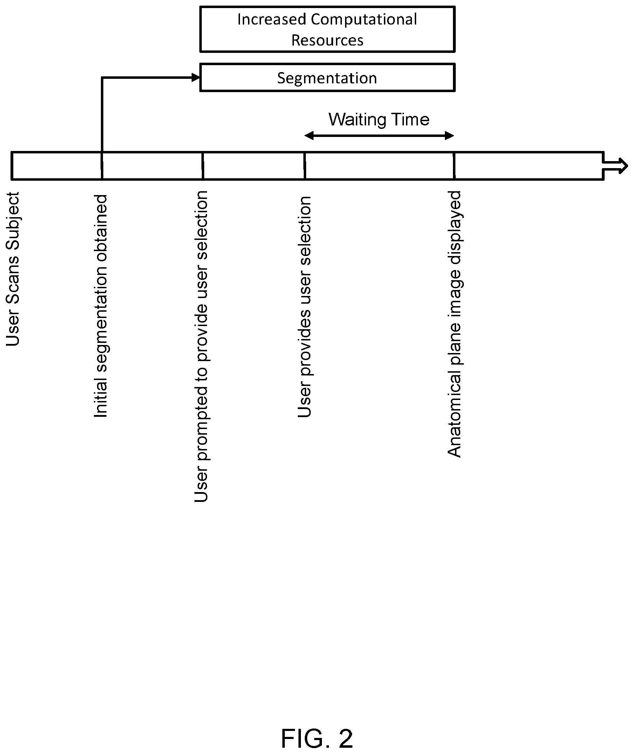

Fig. 2 depicts a workflow of a medical examination, including display of a target anatomical plane image, according to an exemplary embodiment of the invention.

As shown, even before the user is presented with a dialog box for anatomical plane selection, an initial segmentation is performed. One approach would be to reuse a segmentation that has been created at the start of the procedure (e.g., when the user actively creates a dynamic model of an anatomy of the subject) as the initial segmentation, or at least basing the initial segmentation on this segmentation. Alternatively, or additionally, the initial segmentation may be based on segmentation of a previous 3D medical image (to the present 3D medical image to be segmented).

Accordingly, any further adaptations of the segmentation may use this subject-specific segmentation as the starting point of adaptation of the segmentation. As a result, it may take less time to complete the segmentation of the 3D medical image. For example, as it is closer to the subject's shape, fewer iterations may be needed for adaptation.

Then, segmentation of the latest 3D medical image starts not only when the user has selected a target anatomical plane, but already when the user is presented with a dialog box for anatomical plane selection. This gives an extra computation time while the user selects the plane (typically a few hundred milliseconds).

In addition, increased computing resources are allocated to the segmentation algorithm between the user being presented with a dialog box for anatomical plane selection and the completion of the segmentation. This may be achieved by reducing allocation of computational resources to other tasks, for example the actual 3D medical image acquisition. Indeed, the 3D imaging system typically dedicates significant computational resources to acquire the 3D medical image. It has been noticed that it is feasible to reduce some of that computational load whilst having minimal impact on image quality of the 3D medical image. Reducing this computational load would makes computational resources available for performing the segmentation.

In addition, although not depicted, a background task may be run to intermittently establish anatomical context. In the case of the 3D medical image being of the heart, this anatomical context may be established based on the current heartbeat. This anatomical context could be used to display a preliminary approximation of an anatomical plane image directly after the user selects the target anatomical plane (without performing the segmentation to establish anatomical context). The anatomical plane image could then be updated once anatomical context is established from the segmented 3D medical image.

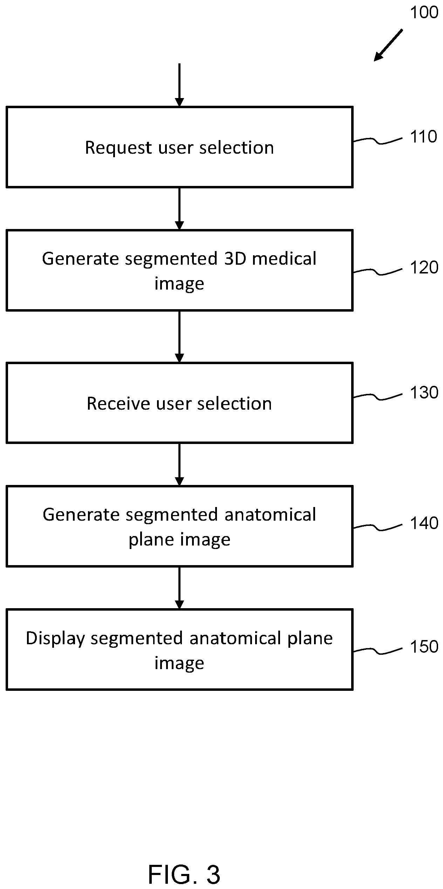

Fig. 3 is a flow diagram of a method for displaying a medical image of a subject according to an embodiment of the invention. The medical image may be displayed during, for example, a scanning session or examination of the subject. In some embodiments, the medical image may be an ultrasound image, but may equally be a CT image, an MRI image, etc.

Initially, the 3D medical image may be obtained. The 3D medical image may be obtained, for example from memory. Alternatively, the 3D medical image may be directly acquired from equipment used to image the subject (e.g., from an ultrasound probe). In some embodiments, a stream of 3D medical images may be received (for example, in real-time as the images are acquired). In this case, the 3D medical image may be the latest 3D medical image in the stream of 3D medical images (or the latest image in the stream of 3D medical images that contains an anatomy of interest).

In step 110, a user selection is requested. The user selection indicates a target anatomical plane of a 3D medical image of the subject.

That is, the user is prompted to provide a user selection. This may be by way of a pop up dialog box, drop down box, etc. In exemplary embodiments, requesting the user selection comprises displaying a plurality of anatomical sketches of target anatomical planes included in the 3D medical image. Of course, the request may be made responsive to the user indicating that they wish to make a user selection, for example by opening a selection menu.

At step 120, the 3D medical image is processed with a segmentation algorithm to generate a segmented 3D medical image.

In practice, the segmentation algorithm may mark or note parts of the 3D medical image that correspond to certain anatomies. In other words, the segmentation algorithm annotates the 3D medical image with anatomical context, thus generating a 3D medical image. For example, in the case that the 3D medical image is of a heart, the 3D medical image may be segmented to indicate parts corresponding to a left ventricle, a right ventricle, left aorta, etc.

The segmentation algorithm may be any known algorithm for segmenting a 3D medical image. For example, the segmentation algorithm may be a model-based segmentation method. That is, a model of an anatomical object is used to provide a-priori knowledge about the shape and appearance of the anatomical object in the 3D medical image. Other algorithms are readily apparent and implementable by the skilled person.

There may be provided a single segmentation algorithm configured to generate a segmented 3D medical image for identification of all possible anatomical planes. That is, the segmentation algorithm may be configured for segmenting a wide range of anatomies, or an expected set of anatomies (e.g., all parts of the heart, or only sub-parts of the heart expected to be present in the 3D medical image).

Alternatively, there may be multiple segmentation algorithms available, each associated with a different subset of anatomies or fields-of-view. That is, there may be a first segmentation algorithm suitable for identifying a first subset of anatomical objects, a second segmentation algorithm suitable for identifying a second subset of anatomical objects, and so on. To this end, each segmentation algorithm may be similar, but may be trained to segment based on a different anatomical model, sub-anatomical model, or a different view of the anatomy. Thus, different field-of-views of the 3D medical image may be more effectively processed by one of the segmentation algorithms that is adapted for the particular field-of-view or image orientation. This may result in a faster and more robust segmentation of the 3D medical image, as long as an appropriate segmentation algorithm is selected for the field-of-view of the 3D medical image.

Accordingly, in the case that multiple segmentation algorithms are available, before receiving the user selection, a segmentation algorithm associated with anatomical objects may need to be identified that is most likely to be needed for segmenting anatomical objects in the target anatomical plane selected by the user. The segmentation algorithm may be selected from a plurality of segmentation algorithms based on, for example: (i) information about the procedure at hand (e.g. mitral valve views for a mitral valve procedure); (ii) a previous selection (most likely that the same model is needed again); (iii) a general user preference. If the segmentation is complete before the user selects a plane, an additional segmentation with the second-most likely model may be run, and so on.

By way of specific example, a target anatomical plane may be a certain mitral valve plane from a 3D medical image showing only the mitral valve, or a certain aortic valve plane from a 3D medical image showing only the aortic valve etc. Therefore, having a particular segmentation algorithm having dedicated models for a specific anatomical features (e.g., the mitral valve or aortic valve) or field-of-view can then result in faster and/or more robust segmentation of the specific field of view.

Optionally, whilst the segmentation algorithm is being used to process the 3D medical image, computational resources available to process the 3D medical image with the segmentation algorithm may be increased. For example, computational resources used to acquire the 3D medical image may be reduced. It has been shown that slightly decreasing the computational resources used to acquire the 3D medical image may have minimal impact on the quality of the 3D medical image, whilst enabling the segmentation algorithm to run much faster.

In step 130, the user selection is received from the user. That is, the user selects a target anatomical plane that they wish to be displayed. This selection may be received as a selection of one of a plurality of standard target anatomical planes. The selection may be by way of a click, or other indication of a desire to view a particular target anatomical plane.

Of course, some time will elapse between the user selection being requested and the user selection being received, as the user will need some time to react and decide upon a target anatomical plane. Accordingly, the step of processing the 3D medical image to generate the segmented 3D medical image begins after requesting the user selection and before receiving the user selection. As a result, time (that would otherwise go unused) is utilized to establish anatomical context of the 3D medical image, that may make it straightforward to generate and display an anatomical plane image. It may be the case that not enough time passes to perform the segmentation before the user provides a user selection. However, the segmentation will be at least partially completed during this time.

In step 140, once the segmented 3D medical image is generated, and the user selection is received, the segmented 3D medical image is processed to generate a segmented anatomical plane image. The segmented anatomical plane image will correspond to the target anatomical plane of the user selection. That is, the segmented anatomical plane image will be a 2D image containing information of the target anatomical plane (e.g., will contain an image of a cross section of an aorta of the subject).

The segmented 3D medical image may be processed in any known manner to generate the (2D) segmented anatomical plane image. This may comprise taking a cross section of cutting of the 3D medical image containing the target anatomical plane.

More specifically, step 140 may comprise identifying a 2D cross section of the segmented 3D medical image including the target anatomical plane of the user selection. This may be particularly straightforward when the 3D medical image has been segmented, as it may be clear where in the 3D medical image the target anatomical plane is. Then, the segmented anatomical plane image is extracted from the segmented 3D medical image based on the identified 2D cross section.

Finally, in step 150, the segmented anatomical plane image is displayed. This may be displayed on a screen for the user to observe, for instance. Due to the time savings associated with performing the segmentation before receiving the user selection, the delay between receiving the user selection and displaying the segmented anatomical plane image may be minimal.

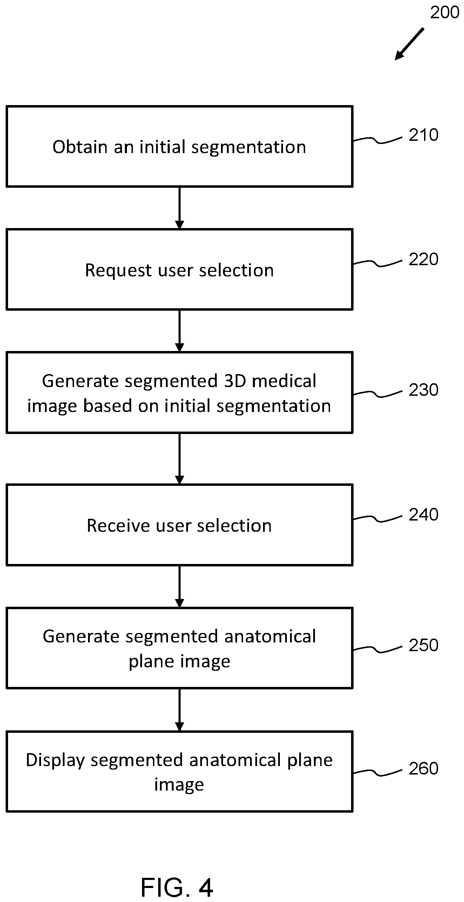

Fig. 4 is a flow diagram of a method 200 for displaying a medical image of a subject according to another embodiment of the invention.

In step 210, there is provided a first step of obtaining an initial segmentation of the 3D medical image. The initial segmentation may be considered an approximate segmentation of the 3D medical image of the subject, upon which the complete segmentation may be based. The initial segmentation may simply be a shape of the anatomy expected to be within the 3D medical image. For example, if the 3D medical image is expected to be of a heart, the initial segmentation may include the shape of an average heart taking into account characteristics of the subject.

Obtaining the initial segmentation may thus comprise simply retrieving the initial segmentation of the subject from a database, or may comprise generating the initial segmentation.

In one embodiment, obtaining the initial segmentation may comprise receiving/obtaining a model of a target anatomical object of the subject. The model may be a basic model of the anatomy describing a static shape of the anatomy, or may be a complex dynamic model that may describe a shape of the anatomy that changes with time (e.g., a model of the heart over a heartbeat).

Accordingly, the model may be used as basis for segmentation of the 3D medical image. That is, because the model provides the shape of the anatomy within the 3D medical image, the shape may only need to be reoriented and/or slightly altered in order to achieved segmentation of the 3D medical image.

In another embodiment, obtaining the initial segmentation comprises receiving one or more previous segmented 3D medical images of the subject.

These previous segmented 3D medical images may be from a historical scan of the subject, or may be segmentations acquired in previous frames of the same scan as the current 3D medical image to be segmented. Furthermore, this step may instead comprise a preceding step (i.e., a step performed before acquiring the present 3D medical image of the subject) of receiving previous (unsegmented) 3D medical images of the subject, and processing, with a segmentation algorithm, the previous 3D medical images to generate one or more previous segmented 3D medical images. In any case, the previous segmented 3D medical image will contain at least a part of the same anatomy as an anatomy present in the 3D medical image.

Then, the initial segmentation may be based on the one or more previous segmented 3D medical images. That is, the initial segmentation may be the same as one of the previous segmented 3D medical images, an average of the previous segmented 3D medical images, or may be an altered version of the previous segmented 3D medical images (e.g., the alteration based on known differences between the current 3D medical image and the previous 3D medical images, such as a zoom factor or a rotation).

In step 220, a user selection indicating a target anatomical plane of a 3D medical image of the subject is requested, similarly to step 110 described above. That is, the user is asked to provide a user selection indicating a target anatomical plane to be displayed.

In step 230, the 3D medical image is processed with a segmentation algorithm to generate a segmented 3D medical image based on the initial segmentation, similarly to step 120 described above. In other words, the 3D medical image and the initial segmentation are provided to the segmentation algorithm, and the segmented 3D medical image is generated. The segmentation algorithm may use the initial segmentation as basis and/or as a starting point for processing the 3D medical image to generate the segmented 3D medical image.

As the initial segmentation is provided to the segmentation algorithm, the segmentation algorithm may be able to generate the segmented 3D medical image faster than when not provided with an initial segmentation. This is because the initial segmentation may provide an approximation for the final segmented 3D medical image, requiring only a few modifications.

In step 240, the user selection is received from the user, similarly to step 130 described above.

In this embodiment, the segmentation of the 3D medical image may be performed before or after receiving the user selection. That is, while the 3D medical image may be processed with the segmentation algorithm before receiving the user selection (thus saving time), the initial segmentation may be obtained and used independently of this feature. Nevertheless, both the initial segmentation and the processing of the 3D medical image with the segmentation algorithm before receiving the user selection, may be implemented by proposed embodiments.

In step 250, the segmented 3D medical image is processed to generate a segmented anatomical plane image corresponding to the target anatomical plane of the user selection. This step may be similar to step 140 described above.

Finally, in step 260, the segmented anatomical plane image is displayed, similar to step 150 described above.

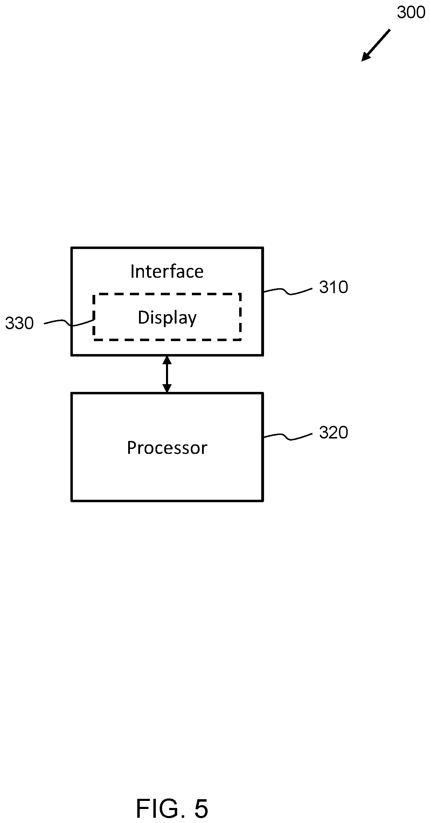

Fig. 5 is a simplified block diagram of a system 300 for displaying a medical image of a subject. The system comprises an interface 310 and a processor 320. The interface 310 may comprise a display 330.

The interface 310 is configured to request a user selection, the user selection indicating a target anatomical plane of a 3D medical image of the subject. The interface 310 is also configured to receive the user selection.

For example, the interface 310 may be any device, component or components capable of outputting information to, and receiving information from, a user. To this end, the interface 310 may be a touch screen, a screen and mouse, a virtual reality peripheral, etc. In some embodiments, the interface 310 comprises a display 330 configured to convey the request for the user selection and/or to receive the user selection.

The processor 320 is configured to process the 3D medical image with a segmentation algorithm in order to generate a segmented 3D medical image. Therefore, the processor 320 may be able to access the segmentation algorithm, either by having the segmentation algorithm installed thereon, retrieving the algorithm from an external device, or by prompting the segmentation to be run on an external device (e.g., on the cloud).

According to embodiment of the invention, the processor 320 is configured to begin processing the 3D medical image with the segmentation algorithm after requesting the user selection and before receiving the user selection. That is, the processor 320 begins generation of the segmented 3D medical image after the interface 310 requests the user selection, but before the user interface 310 receives the user selection.

In additional or alternative embodiments of the invention, the processor 320 is configured to obtain an initial segmentation of the 3D medical image. In this case, the processor 320 is configured to process the 3D medical image with the segmentation algorithm to generate a segmented 3D medical image based on the initial segmentation. Of course, the processor 320 may be configured to obtain the initial segmentation of the 3D medical image according to any disclosed method.

Furthermore, the processor 320 may also be configured to increase computational resources available to process the 3D medical image with the segmentation algorithm. That is, the processor 320 may allocate additional computational resources to process the 3D medical image when the segmentation is to be performed. This may be achieved, for example, by reducing computational resources allocated for acquiring 3D medical images.

In addition, the processor 320 is also configured to process the segmented 3D medical image to generate a segmented anatomical plane image corresponding to the target anatomical plane of the user selection.

Finally, the interface 310 is further configured to display 330 the segmented anatomical plane image. This may be achieved, for example, by outputting to the display 330. Of course, the interface 310 may instead transmit the segmented anatomical plane image to a separate display, monitor or other means for displaying an image.

In any case, the proposed system 300 reduces a time between receiving a user selection, and displaying the segmented anatomical plane image by reducing a length of time taken to segment the 3D medical image during this period of time.

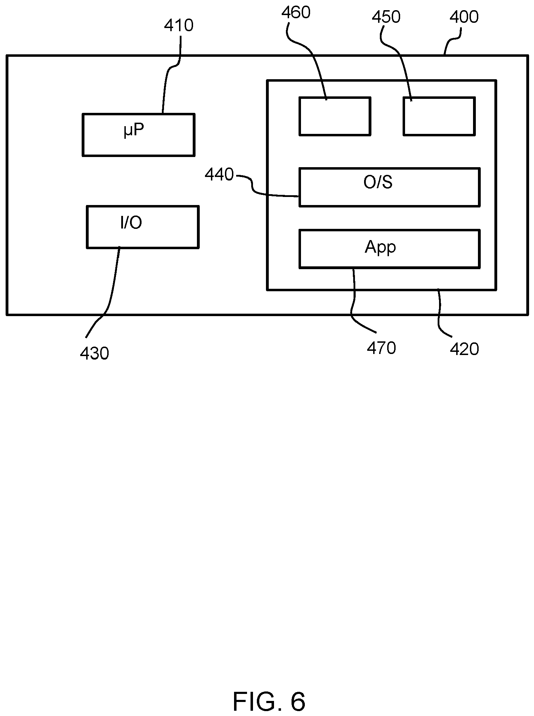

Fig. 6 illustrates an example of a computer 410 within which one or more parts of an embodiment may be employed. Various operations discussed above may utilize the capabilities of the computer 410. For example, one or more parts of a system for controlling a handheld device may be incorporated in any element, module, application, and/or component discussed herein. In this regard, it is to be understood that system functional blocks can run on a single computer or may be distributed over several computers and locations (e.g. connected via internet), such as a cloud-based computing infrastructure.

The computer 410 includes, but is not limited to, PCs, workstations, laptops, PDAs, palm devices, servers, storages, microcontroller units, integrated processors, AI-accelerators, and the like. Generally, in terms of hardware architecture, the computer 410 may include one or more processors 410, memory 420, and one or more I/O devices 430 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 410 is a hardware device for executing software that can be stored in the memory 420. The processor 410 can be virtually any custom made or commercially available processor, a central processing unit (CPU), a digital signal processor (DSP), a tensor processing unit (TSP) specifically designed for neural processing, a dedicated AI accelerator/processing unit or an auxiliary processor among several processors associated with the computer 410, and the processor 410 may be a semiconductor based microprocessor (in the form of a microchip) or a microprocessor.

The memory 420 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 420 may incorporate electronic, magnetic, optical, and/or other types of storage media. Note that the memory 420 can have a distributed architecture, where various components are situated remote from one another, but can be accessed by the processor 410.

The software in the memory 420 may include one or more separate programs, each of which comprises an ordered listing of executable instructions for implementing logical functions. The software in the memory 420 includes a suitable operating system (O/S) 440, compiler 460, source code 450, and one or more applications 470 in accordance with exemplary embodiments. As illustrated, the application 470 comprises numerous functional components for implementing the features and operations of the exemplary embodiments. The application 470 of the computer 410 may represent various applications, computational units, logic, functional units, processes, operations, virtual entities, and/or modules in accordance with exemplary embodiments, but the application 470 is not meant to be a limitation.

The operating system 440 controls the execution of other computer programs, and provides scheduling, input-output control, file and data management, memory management, and communication control and related services. It is contemplated by the inventors that the application 470 for implementing exemplary embodiments may be applicable on all commercially available operating systems.

Application 470 may be a source program, executable program (object code), script, or any other entity comprising a set of instructions to be performed. When a source program, then the program is usually translated via a compiler (such as the compiler 460), assembler, interpreter, or the like, which may or may not be included within the memory 420, so as to operate properly in connection with the O/S 440. Furthermore, the application 470 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, NET, and the like.

The I/O devices 430 may include input devices such as, for example but not limited to, a mouse, keyboard, scanner, microphone, camera, etc. Furthermore, the I/O devices 430 may also include output devices, for example but not limited to a printer, display, etc. Finally, the I/O devices 430 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 430 also include components for communicating over various networks, such as the Internet or intranet.

If the computer 410 is a PC, workstation, intelligent device or the like, the software in the memory 420 may further include a basic input output system (BIOS) (omitted for simplicity). The BIOS is a set of essential software routines that initialize and test hardware at startup, start the O/S 440, and support the transfer of data among the hardware devices. The BIOS is stored in some type of read-only-memory, such as ROM, PROM, EPROM, EEPROM or the like, so that the BIOS can be executed when the computer 410 is activated.

When the computer 410 is in operation, the processor 410 is configured to execute software stored within the memory 420, to communicate data to and from the memory 420, and to generally control operations of the computer 410 pursuant to the software. The application 470 and the O/S 440 are read, in whole or in part, by the processor 410, perhaps buffered within the processor 410, and then executed.

When the application 470 is implemented in software it should be noted that the application 470 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 470 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

The proposed control method(s) of Figs. 3 and 4, and the system(s) of Fig. 5, may be implemented in hardware or software, or a mixture of both (for example, as firmware running on a hardware device). To the extent that an embodiment is implemented partly or wholly in software, the functional steps illustrated in the process flowcharts may be performed by suitably programmed physical computing devices, such as one or more central processing units (CPUs) or graphics processing units (GPUs). Each process - and its individual component steps as illustrated in the flowcharts - may be performed by the same or different computing devices. According to embodiments, a computer-readable storage medium stores a computer program comprising computer program code configured to cause one or more physical computing devices to carry out a control method as described above when the program is run on the one or more physical computing devices.

Storage media may include volatile and non-volatile computer memory such as RAM, PROM, EPROM, and EEPROM, optical discs (like CD, DVD, BD), magnetic storage media (like hard discs and tapes). Various storage media may be fixed within a computing device or may be transportable, such that the one or more programs stored thereon can be loaded into a processor.

To the extent that an embodiment is implemented partly or wholly in hardware, some of the blocks shown in the block diagrams of Fig 5. may be separate physical components, or logical subdivisions of single physical components, or may be all implemented in an integrated manner in one physical component. The functions of one block shown in the drawings may be divided between multiple components in an implementation, or the functions of multiple blocks shown in the drawings may be combined in single components in an implementation. Hardware components suitable for use in embodiments of the present invention include, but are not limited to, conventional microprocessors, application specific integrated circuits (ASICs), and field-programmable gate arrays (FPGAs). One or more blocks may be implemented as a combination of dedicated hardware to perform some functions and one or more programmed microprocessors and associated circuitry to perform other functions.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

The flowchart and block diagrams in the Figures illustrate the architecture, functionality, and operation of possible implementations of systems, methods, and computer program products according to various embodiments of the present invention. In this regard, each block in the flowchart or block diagrams may represent a module, segment, or portion of instructions, which comprises one or more executable instructions for implementing the specified logical function(s). In some alternative implementations, the functions noted in the block may occur out of the order noted in the Figures. For example, two blocks shown in succession may, in fact, be executed substantially concurrently, or the blocks may sometimes be executed in the reverse order, depending upon the functionality involved. It will also be noted that each block of the block diagrams and/or flowchart illustration, and combinations of blocks in the block diagrams and/or flowchart illustration, can be implemented by special purpose hardware-based systems that perform the specified functions or acts or carry out combinations of special purpose hardware and computer instructions.

## Claims

1. A method for displaying a medical image of a subject, comprising:
requesting (110) a user selection, the user selection indicating a target anatomical plane of a 3D medical image of the subject;
processing, with a segmentation algorithm, the 3D medical image to generate (120) a segmented 3D medical image;
receiving (130) the user selection;
processing the segmented 3D medical image to generate (140) a segmented anatomical plane image corresponding to the target anatomical plane of the user selection; and
displaying (150) the segmented anatomical plane image,
wherein the step of processing the 3D medical image begins after requesting the user selection and before receiving the user selection.

2. The method of claim 1, further comprising a preceding step of obtaining (310) an initial segmentation of the 3D medical image, and wherein processing the 3D medical image to generate (120) the segmented 3D medical image is further based on the initial segmentation of the 3D medical image.

3. The method of claim 2, wherein obtaining (310) the initial segmentation comprises:
receiving a model of a target anatomical object of the subject; and
processing, with the segmentation algorithm, the 3D medical image and the model to generate the initial segmentation.

4. The method of claim 2 or 3, wherein obtaining (310) the initial segmentation comprises:
receiving one or more previous segmented 3D medical images of the subject;
generating the initial segmentation based on the one or more previous segmented 3D medical images.

5. The method of any preceding claim, further comprising receiving a stream of 3D medical images, and wherein the 3D medical image is a most recent 3D medical image in the stream of 3D medical images.

6. The method of claim 5, wherein the stream of 3D medical images is received in real-time.

7. The method of any of claims 1-6, further comprising increasing computational resources available to process the 3D medical image with the segmentation algorithm.

8. The method of any of claims 1-7, wherein processing the segmented 3D medical image to generate (120) the segmented anatomical plane image comprises:
identifying a 2D cross section of the segmented 3D medical image including the target anatomical plane of the user selection; and
extracting the segmented anatomical plane image from the segmented 3D medical image based on the identified 2D cross section.

9. The method of any of claims 1-8, wherein requesting (110) the user selection comprises displaying a plurality of anatomical sketches of target anatomical planes included in the 3D medical image, and wherein receiving the user selection comprises receiving a selection of one of the anatomical sketches from the user.

10. The method of any of claims 1-9, further comprising scanning the subject to acquire the 3D medical image.

11. The method of any of claims 1-10, wherein the 3D medical image comprises a 3D ultrasound image of the subject.

12. A computer program comprising computer program code means adapted, when said computer program is run on a computer, to implement the method of any of claim 1-11.

13. A system (300) for displaying a medical image of a subject, comprising:
an interface (310) configured to:
request a user selection, the user selection indicating a target anatomical plane of a 3D medical image of the subject; and
receive the user selection; and
a processor (320) configured to:
process, with a segmentation algorithm, the 3D medical image to generate a segmented 3D medical image;
process the segmented 3D medical image to generate a segmented anatomical plane image corresponding to the target anatomical plane of the user selection,
wherein the processor is configured to begin processing the 3D medical image after the interface requests the user selection and before the interface receives the user selection, and
wherein the interface is further configured to display the segmented anatomical plane image.

14. The system of claim 13, wherein the processor (320) is further configured to:
obtain an initial segmentation of the 3D medical image; and
process, with the segmentation algorithm, the 3D medical image to generate a segmented 3D medical image further based on the initial segmentation of the 3D medical image.

15. The system of claim 13 or 14, wherein the processor (320) is further configured to increase computational resources available to process the 3D medical image with the segmentation algorithm.
